# EUROPEAN PATENT SPECIFICATION

(11) **EP 3 946 016 B1**
(45) Date of publication and mention of the grant of the patent: **30.10.2024**
(21) Application number: 20778121.2
(22) Date of filing: 23.03.2020
(51) Int. Cl.: A61B 5/00, A61B 5/16, A61B 5/01

(54) **APPARATUS FOR MEASURING VIBROTACTILE PERCEPTION AND PREPARATION METHOD THEREOF INCLUDING AUTOMATED MEASUREMENT OF TEMPERATURE**
VORRICHTUNG ZUR MESSUNG DER VIBROTAKTILEN WAHRNEHMUNG UND HERSTELLUNGSVERFAHREN DAFÜR MIT AUTOMATISIERTER MESSUNG DER TEMPERATUR
APPAREIL DE MESURE DE PERCEPTION VIBROTACTILE ET SON PROCÉDÉ DE PRÉPARATION COMPRENANT UNE MESURE AUTOMATISÉE DE LA TEMPÉRATURE

(30) Priority: 27.03.2019 SE 1950373
(43) Date of publication of application: 09.02.2022
(73) Proprietor: Vibrosense Dynamics AB, 205 12 Malmö (SE)
(72) Inventor: OLDE, Bo, 224 67 LUND (SE); SCARSÉN, Michael, 238 36 OXIE (SE); SPEIDEL, Antonio, 218 71 TYGELSJÖ (SE)
(74) Representative: Neij & Lindberg AB
(86) International application number: PCT/SE2020/050298
(87) International publication number: WO 2020/197473

(56) References cited:
- EP-A2- 1 749 588
- WO-A1-00/59377
- WO-A1-2006/046901
- WO-A1-2017/216812
- WO-A1-2017/216812
- JP-A- H05 215 376
- US-A- 5 002 065
- US-A1- 2003 152 133
- US-A1- 2007 191 730
- US-A1- 2007 293 784
- US-A1- 2009 163 783
- US-A1- 2011 313 314
- US-A1- 2012 109 003
- US-A1- 2012 220 892
- US-A1- 2014 163 394
- MINU SHIKHA GANDHI ED - GOLDMAN SARAH B ET AL: "Progress in Vibrotactile Threshold Evaluation Techniques: A Review", JOURNAL OF HAND THERAPY, vol. 24, no. 3, 1 September 2011 (2011-09-01), pages 240 - 256, XP028252445, ISSN: 0894-1130, [retrieved on 20110119], DOI: 10.1016/J.JHT.2011.01.001

## Description

### Technical Field

The present invention relates to techniques for measuring vibrotactile perception in body parts of human test subjects.

### Background Art

Adequate vibrotactile perception in peripheral body parts such as hands and feet is crucial for daily life. Individuals with compromised sensitivity to vibrations in their hands may have difficulty handling small objects, be prone to drop objects or even be incapable of performing everyday tasks such as dressing/undressing, taking care of hygiene or preparing food. Reduced sensitivity in the feet may affect an individual's balance and may impair the individual's ability to perceive formation of blisters on the feet, potentially allowing the blisters to progress into foot ulcers.

Reduced vibrotactile perception may be caused by damage to nerves or blood vessels as a result of mechanical vibrations at high magnitude and long duration. Such mechanical vibrations may e.g. be generated by hand-held vibrating instruments or tools. It is also well-known that impaired vibrotactile perception may result from progressive deterioration of nerve functionality in patients with diabetes, as well as chemically induced neuropathy in patients on chemotherapy.

Irrespective of origin, early detection of peripheral neuropathy is essential to prevent permanent damage or at least prevent a worsening of the condition. Sensitive and accurate measurement of the vibrotactile perception in body parts of human subjects is considered a valuable tool for early and reliable detection of compromised nerve functionality.

In clinical practice, problems with tactile perception are often diagnosed by self-reporting in questionnaires. This is a fast and cheap diagnostic technique. However, even if careful instructions are given to the subject, the technique is entirely subjective and prone to misunderstanding and psychological bias such as the subject's current mood or unwillingness to admit any problem.

Other known diagnostic techniques make use of a monofilament or a tuning fork which is pressed to the skin of the subject to cause a defined force/vibration. The subject is asked to report when the force/vibration is perceived, which indicates the level of tactile sensitivity. This technique is reliant on the skill of the operator and is not suitable for objective and accurate determination.

It is also known to use a specialized medical device known as a vibrometer, biothesiometer, pallesthesiometer or neurothesiometer. This medical device is configured to measure the vibration/vibrotactile perception threshold (VPT) at one or more vibration frequencies. The device comprises a projecting cylinder or probe which, when engaged by a body part of the subject, is controlled to vibrate at varying amplitudes for one or more distinct frequencies. During this test procedure, the subject may be instructed to indicate when the vibration is no longer discernible as the amplitude is gradually decreased, and to indicate when the vibration is sensed again as the amplitude is gradually increased. The amplitudes when perception is lost and regained form VPTs which may be analyzed to quantify the subject's vibrotactile perception.

An example of such a vibrometer is disclosed in WO2006/046901. For quantitative analysis, it is important that the subject applies the body part onto the probe in a consistent manner during the test procedure. To this end, the vibrometer may include a pressure/force sensor which is operatively connected to the probe to measure the application force. The vibrometer provides feedback to the subject or an operator about the measured application force and thereby enables the subject to make adjustments to attain a sufficiently consistent application force on the probe.

It is known that the skin temperature has an impact on the VPTs measured by a vibrometer. For example, the sensitivity in fingers to vibrations has been found to be significantly lower when the fingers are colder than 27°C, see e.g. "Effect of individual finger skin temperature on vibrotactile perception threshold" by Harazin et al, published in Int J Occup Med Environ Health, 2013, 26(6), pp 930-939. It is therefore important to know the skin temperature in order to avoid incorrect or misleading measurements of VPTs. In fact, the ISO standard for vibrotactile perception measurements, given by ISO 13091-1 and 13091-2, stipulate that the skin temperature should be in the range of 27-35°C and that the ambient temperature should be in the range of 20-30°C during the test procedure. The standard also stipulates that the test procedure should be repeated if deviations from these temperature ranges are detected or suspected during or after the test procedure.

WO2006/046901 proposes to mount a temperature sensor on the probe to measure the skin temperature during or at least at the beginning of the test procedure. However, it is technically demanding to integrate a temperature sensor with the probe without affecting its vibrations. Further, the vibrations may affect the durability of the sensor.

In practice, e.g. in the Applicant's commercially available first-generation VibroSense Meter^{®}, the vibrometer housing may include a contact sensor for temperature measurement at a location well-separated from the probe. The contact sensor may be used for measurement of both skin temperature and ambient (room) temperature. Before the test procedure, the subject places the related body part on the contact sensor to check the skin temperature. This additional step takes time and makes the vibrometer more complex to use. To avoid that the initial skin temperature measurement is forgotten, the vibrometer needs to implement a dedicated confirmation step. Further, inadequate contact pressure and contact time between the body part and the contact sensor may compromise the accuracy of the temperature measurement. Also, the measurement of ambient temperature may be biased by the temperature of the vibrometer, which may be heated by the electronics inside the housing. Further relevant documents are US 2007/191730 A1 and WO 2017/216812 A1.

### Summary

It is an objective of the invention to at least partly overcome one or more limitations of the prior art.

A further objective is to facilitate automated measurement of skin temperature of a relevant body part in an apparatus for measuring vibrotactile perception.

Another objective is to enable automated measurement of ambient temperature.

Yet another objective is to ensure the accuracy of the measured temperature(s).

One or more of these objectives, as well as further objectives that may appear from the description below, are at least partly achieved by an apparatus, a method and a computer-readable medium in accordance with the independent claims, embodiments thereof being defined by the dependent claims.

A first aspect of the invention is an apparatus according to claim 1 configured to perform a test procedure for measuring vibrotactile perception of a subject. The apparatus comprises: a vibration probe with an end portion arranged for engagement by a body part of the subject; a contactless temperature sensor arranged to face the body part when engaged with the end portion, the contactless temperature sensor configured to provide a first output signal; a force sensor coupled to the vibration probe, said force sensor configured to provide a second output signal indicative of an application force onto the end portion; and a control unit configured to, before the test procedure, compute two or more characteristic parameters of the first and second output signals indicative of presence or absence of the body part and, when presence of the body part is determined based on the two or more characteristic parameters, set a current temperature of the body part to a current temperature value given by the first output signal.

The apparatus includes a contactless sensor for sensing temperature. Such a contactless sensor is capable of remotely sensing the temperature within a field of view. If the field of view is unobstructed, the sensor measures the temperature of the surroundings, i.e. the ambient temperature. If an object is placed in the field of view, the sensor measures the surface temperature of the object. Since the body part temperature and the ambient temperature are unknown and may overlap, as explained in the Background section, implementation of a contactless temperature sensor in an apparatus for measuring vibrotactile perception requires a technique of discriminating between measurements of ambient temperature and measurements of body part temperature (skin temperature). While this may be achieved by temporal separation of the two types of measurements, e.g. by instructing the subject to place the body part over the sensor at a specific time point, such an additional step takes time, makes the apparatus more complex to use, and is prone to human error. The first aspect is based on the insight that it is desirable to arrange the contactless sensor to face the body part when the body part engages the end portion of the vibration probe, since this would allow for automated measurement of skin temperature when the body part is in place on the probe in advance of the test procedure. The contactless sensor is preferably arranged to measure the temperature close to the area of contact between the body part and the end portion, e.g. within a distance of 0-50 mm from the area of contact. The first aspect is also based on the insight that the above-mentioned temporal discrimination may be replaced by a discrimination based on signal analysis of at least one of the output signal of the temperature sensor and the output signal of a force sensor coupled to the vibration probe, and that such a discrimination enables automated and reliable measurement of skin temperature. Thus, skin temperature may be measured when the subject is preparing for the vibrotactile perception measurement by placing the body part in contact with the end portion of the vibration probe, and without requiring any changes to standard procedures and even without informing or instructing the subject in respect of the temperature measurement. For example, the apparatus may be capable of verifying that the skin temperature falls within the requisite range while it verifies that the application force is adequate.

It is realized that the first aspect facilitates automated measurement of skin temperature by enabling temperature measurement while the body part is engaged with the vibration probe in preparation for a test procedure. The first aspect thereby reduces the preparation time for the test procedure and obviates the risk of forgetting to measure and verify the skin temperature. The first aspect also obviates the need for instructing the subject to measure the skin temperature. As a beneficial side effect, the first aspect also enables automated measurement of skin temperature during the course of the test procedure by use of the contactless sensor. It is also conceivable to verify that the body part is present on the probe during the test procedure based on the output signal of the contactless sensor.

Further, the first aspect ensures the accuracy of the measured skin temperature. Since there is no physical contact between the contactless sensor and the body part, the accuracy is not affected by variations in contact pressure or contact time. Further, variations in measurement time may be minimized since the skin temperature is measured while the body part is engaged with the vibration probe in preparation for the test procedure.

Still further, the contactless sensor may also be operated to measure the ambient temperature. Compared to a contact sensor, the accuracy of the ambient temperature may be improved since the contactless sensor generally is less sensitive to its own temperature and the temperature at the mounting site. A contactless sensor may be configured to measure and compensate for the temperature at the mounting location when determining the temperature within the predefined field of view.

In the following, various embodiments of the first aspect are defined. These embodiments provide at least some of the technical effects and advantages described in the foregoing, as well as additional technical effects and advantages as readily understood by the skilled person, e.g. in view of the following detailed description.

The control unit is further configured to, when absence of the body part on the end portion is determined based on the two or more characteristic parameters, set a current ambient temperature to the current temperature value given by the first output signal.

The two or more characteristic parameters comprises at least two characteristic parameters, wherein the control unit is configured to set the current temperature of the body part to the current temperature value given by the first output signal, only when all of the characteristic parameters indicate presence of the body part.

The control unit is further configured to, when at least one of the two or more characteristic parameters indicate absence of the body part on the end portion, set a current ambient temperature to the current temperature value given by the first output signal.

In some embodiments, the contactless temperature sensor is an electromagnetic radiation sensor.

The control unit is configured to compute the two or more characteristic parameters as a function of both the first output signal and the second output signal.

In some embodiments, the control unit is configured to determine presence of the body part on the end portion by evaluating the two or more characteristic parameters in relation to a respective range of values.

In some embodiments, the control unit is configured to compute at least one of the two of more characteristic parameters to represent a temporal gradient in the first or second output signal, and evaluate the temporal gradient in relation to a range of gradient values for determination of presence of the body part on the end portion.

In some embodiments, the control unit is configured to compute at least one of the two of more characteristic parameters to represent a variability in the first or second output signal, and evaluate the variability in relation to a variability reference for determination of presence of the body part on the end portion.

In some embodiments, the control unit is configured to, during a reference phase in which the body part is absent from the end portion, compute a baseline temporal variability, and set the variability reference based on the baseline temporal variability.

In some embodiments, the apparatus further comprises a heat source which is arranged to generate heat at the body part, if the body part is engaged with the end portion, for detection by the contactless temperature sensor, the control unit being further configured to cause the heat source to be activated at least once in a predefined time period and compute at least one of the characteristic parameters in the first output signal during the predefined time period.

In some embodiments, the control unit is further configured to, when absence of the body part is determined based on the two or more characteristic parameters, set a current ambient temperature to the current temperature value given by the first output signal when the heat source is deactivated.

In some embodiments, the control unit is further configured to compute at least one of the two of more characteristic parameters to represent a magnitude in the first or second output signal, and evaluate the magnitude in relation to a magnitude range for determination of presence of the body part on the end portion.

In some embodiments, the apparatus comprises a further temperature sensor, and the control unit is configured to obtain a temperature value from the further temperature sensor and set the magnitude range based on the temperature value.

In some embodiments, the apparatus comprises a housing containing at least the vibration probe and the contactless temperature sensor, and the further temperature sensor is located to sense a temperature inside the housing.

In some embodiments, the two or more characteristic parameters represent at least one of a magnitude, a variability and a temporal gradient in the first and/or second output signal.

In some embodiments, the apparatus further comprises: a support surface portion which is spaced from the vibration probe, and a contact sensor arranged at the support surface portion to detect contact between the body part and the support surface portion, wherein the control unit is configured to: obtain a third output signal from the contact sensor, and set the current temperature of the body part to the current temperature value given by the first output signal only when presence of the body part is determined based on the one or more characteristic parameters and the third output signal indicates said contact between the body part and the support surface portion.

In some embodiments, the apparatus further comprises a housing that defines a first opening and a second opening, which is spatially separate from the first opening, and the vibration probe is arranged in the housing in alignment with the first opening, and the contactless temperature sensor is arranged in the housing in alignment with the second opening.

A second aspect is a method according to claim 10 of preparing an apparatus for measuring vibrotactile perception of a subject, the apparatus comprising a vibration probe having an end portion arranged for engagement by a body part of the subject. The method comprises: obtaining a first output signal from a contactless temperature sensor arranged to face the body part when the body part is engaged with the end portion of the vibration probe, and obtaining a second output signal from a force sensor coupled to the vibration probe, said second output signal being indicative of an application force onto the end portion; computing two or more characteristic parameters of the first and second output signals; evaluating the two or more characteristic parameters for presence of the body part; and setting, when said evaluating indicates presence of the body part, a current temperature of the body part to a current temperature value given by the first output signal.

Any one of the embodiments of the first aspect may be adapted and implemented as an embodiment of the second aspect.

A third aspect of the invention is a computer-readable medium according to claim 11 comprising computer instructions which, when executed by a processor, cause the processor to perform the method of the second aspect or any of its embodiments.

A non claimed fourth aspect is an apparatus configured to perform a test procedure for measuring vibrotactile perception of a subject. The apparatus comprises: a vibration probe with an end portion arranged for engagement by a body part of the subject; a contactless temperature sensor configured to provide a first output signal and arranged with its field of view facing the body part when the body part is engaged with the end portion; a presence sensor configured to provide a second output signal indicative of presence of the body part in the field of view; and a control unit configured to, before the test procedure, evaluate the second output signal for presence of the body part in the field of view and, when presence of the body part is determined, set a current temperature of the body part to a current temperature value given by the first output signal. In one embodiment, the presence sensor is a radiation detector which is configured to detect a shadow of the body part. In one embodiment, a field of view of the radiation sensor at least partly overlaps the field of view of the contactless temperature sensor. In one embodiment, the presence sensor is configured to at least partly detect radiation in a visible wavelength range.

A non claimed fifth aspect is a method of preparing an apparatus for measuring vibrotactile perception of a subject, the apparatus comprising a vibration probe having an end portion arranged for engagement by a body part of the subject. The method comprises: obtaining a first output signal from a contactless temperature sensor arranged with its field of view facing the body part when the body part is engaged with the end portion; obtaining a second output signal from a presence sensor which is responsive to presence of the body part in the field of view; evaluating the second output signal for presence of the body part in the field of view; and setting, when said evaluating determines presence of the body part, a current temperature of the body part to a current temperature value given by the first output signal.

Still other objectives, features, embodiments, aspects and advantages of the present invention may appear from the following detailed description, from the attached claims as well as from the drawings.

### Brief Description of the Drawings

Embodiments of the invention will now be described in more detail with reference to the accompanying drawings.
FIG. 1 is a schematic side view, partly in section, of an apparatus for measuring vibrotactile perception of a body part in accordance with an embodiment.
FIG. 2 is a graph of an exemplifying output signal from a sensor in the apparatus of FIG. 1 during engagement of the body part with the apparatus.
FIG. 3 is a flow chart of a process for temperature determination in accordance with an embodiment.
FIGS 4A-4F are flow charts of processes for temperature determination in accordance with further embodiments.
FIG. 5 is a schematic side view of an apparatus for measuring vibrotactile perception of a body part in accordance with a further embodiment.
FIG. 6 is a flow chart of a process for temperature determination in accordance with a further embodiment.

### Detailed Description of Example Embodiments

Embodiments of the present invention will now be described more fully hereinafter with reference to the accompanying drawings, in which some, but not all, embodiments of the invention are shown. Indeed, the invention may be embodied in many different forms and should not be construed as limited to the embodiments set forth herein; rather, these embodiments are provided so that this disclosure may satisfy applicable legal requirements. Like numbers refer to like elements throughout.

Also, it will be understood that, where possible, any of the advantages, features, functions, devices, and/or operational aspects of any of the embodiments of the present invention described and/or contemplated herein may be included in any of the other embodiments of the present invention described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention. Similarly, the expressions "as a function of" and "based on" in combination with a specified set of parameters or the like are inclusive and do not to preclude the presence or addition of further parameters.

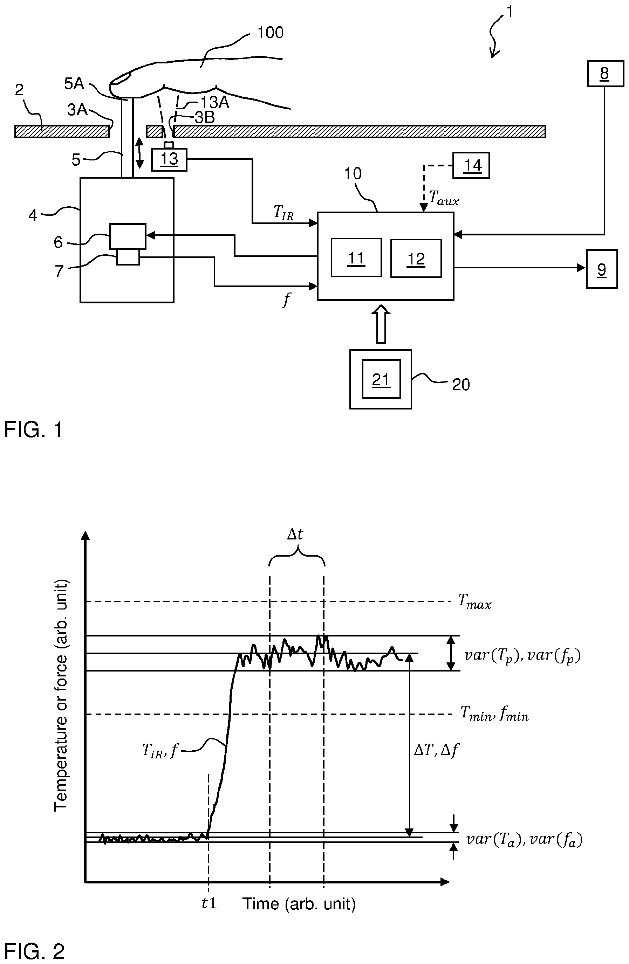

FIG. 1 is a schematic section view of an apparatus 1 for measuring vibrotactile perception, denoted "vibrometer" in the following. The vibrometer 1 is configured to determine vibration perception thresholds, VPTs, at one or more frequencies for a body part 100 of a human subject to be tested ("test subject"). Techniques for determining and evaluating VPTs are well-known in the art and will not be described in detail herein. The VPTs may be measured for any body part 100, including a hand or a foot. In the illustrated example, the body part 100 is a hand, and the vibrotactile perception is measured for a finger on the hand.

The vibrometer 1 comprises a housing 2, only shown in part, which encloses an electro-mechanical measurement system and defines first and second openings 3A, 3B. The housing 2 may also serve as a mechanical support for the body part 100 to improve the comfort of the test subject and to ensure that the VPTs are measured in a consistent and repeatable way. The measurement system includes a measurement device 4 with a vibration probe 5 which is arranged to project through the first opening 3A to expose the distal probe end 5A ("end portion"). The measurement device 4 includes a vibrator 6, which is an electro-dynamic device that vibrates when supplied with a current or voltage, and a force sensor 7. The vibration probe 5 is coupled to the vibrator 6 which is thereby operable to impart a longitudinal vibration to the probe 5, as indicated by a double-ended arrow. The force sensor 7 is directly or indirectly coupled to the probe 5 to sense the longitudinal application force onto the probe end 5A. The force sensor 7 may be of any type, and the force may be given by direct force measurement, e.g. by one or more strain gauges, accelerometers, etc., or by indirect measurement of displacement of the probe or the vibrator 6. The force may also be indirectly given by analysis of the supply current to the vibrator 6. The vibrometer 1 further includes an input device 8 which allows the test subject to signal when the vibration of the probe 5 is sensed by the test subject. The input device 8 may include any combination of a button, a keyboard, a key pad, a microphone, a gesture recognition system, etc. A feedback device 9 enables the vibrometer 1 to output indications or instructions to the test subject or an operator of the system 1. The feedback device 9 may include any combination of indicator lamp, display, touch screen, speaker, etc. A control unit 10 operates the vibrometer 1 and is connected, by wire or wirelessly, to output a control signal to the vibrator 6 and a feedback signal to the feedback device 9 and to receive a force signal *f* from the force sensor 7 and an input signal from the input device 8. The control unit 10 is configured to perform a test procedure to obtain VPTs. During the test procedure, the control unit 10 operates the vibrator 6 to impart vibrations to the probe 5 at different intensities (amplitudes) at one or more frequencies. The test subject, which has engaged a finger, e.g. the fingertip as shown, with the probe end 5A, then indicates via the input device 8 when the vibration of the probe 5 is sensed. During the test procedure, the control unit 10 monitors the force signal *f* from the sensor 7 to ensure that the application force of the fingertip on the probe end 5A is adequate.

In the illustrated example, the control unit 10 comprises a processor 11 and computer memory 12. A control program comprising program instructions is stored in the memory 12 and executed by the processor 11 to control the operation of the vibrometer 1, including all methods and processes disclosed herein. As indicated, the control program 21 may be supplied to the control unit 10 on a computer-readable medium 20, which may be a tangible (non-transitory) product (e.g. magnetic medium, optical disk, read-only memory, flash memory, etc.) or a propagating signal.

In FIG. 1, the vibrometer 1 further includes a contactless temperature sensor 13, which is arranged in alignment with the second opening 3B to measure the temperature within a field view 13A, indicated by dashed lines in FIG. 1. In an alternative (not shown), the sensor 13 is arranged in the first opening 3A and the second opening 3B is omitted. The provision of separate openings 3A, 3B may facilitate manufacture and proper placement of the field of view 13A of the sensor 13. Further, the two separate openings may be designed with a smaller total area than a single opening accommodating both probe 5 and sensor 13, thereby reducing the risk for ingress of contaminants, dust, solid objects, moisture, etc. The sensor 13 is arranged such that the field of view 13A is located adjacent to the probe end 5A. In this context, "located adjacent to" implies that the field of view 13A may be spaced from, border on or include the probe end 5A. The contactless sensor 13 ("remote sensor") may be of any type that enables remote measurement of temperature, including different types of electromagnetic radiation sensors, e.g. operating in the infrared or near-infrared spectrum. Such remote sensors include bolometers, thermopile sensors, pyroelectric detectors, optical filament pyrometers, quantum detectors, and thermochromic sensors. The control unit 10 is connected, by wire or wirelessly, to receive a temperature signal *T_{IR}* from the sensor 13. In some embodiments, described further below, the control unit 10 may also be connected to receive an auxiliary temperature signal *Tₐᵤₓ* from a second temperature sensor 14, which is arranged to sense the ambient temperature inside the vibrometer 1, i.e. within the housing 2. The second sensor 14 may be of any conceivable type and may be integrated with the sensor 13. In one embodiment, the control unit 10 or the sensor 13 is configured to use the signal *Tₐᵤₓ* to compensate the signal *T_{IR}* for the local temperature at the sensor 13, as known in the art.

As understood from the Background section, it is desirable to verify the skin temperature of the body part 100, and preferably also the surrounding (ambient) temperature at the location of the vibrometer 1, before initiating the test procedure. It is also preferable that such verification is made automatically by the vibrometer 1 and without imposing constraints or special actions on the test subject. The temperature signal *T_{IR}* of the sensor 13 will indicate the average temperature within the field of view 13A. It is realized that the average temperature will correspond to the ambient temperature when the body part 100 is not located within the field of view 13A, and the skin temperature when the body part 100 is located within the field of view 13A. However, since the skin temperature and the ambient temperature may attain similar values, it is challenging to discriminate between ambient and skin temperature in the temperature signal *T_{IR}.* Embodiments of the invention seek to overcome this problem, and preferably with minimum increase in cost and complexity of the vibrometer 1.

FIG. 2 depicts a measurement signal as a function of time and may be seen to schematically represent either the force signal *f* from sensor 7 or the temperature signal *T_{IR}* from sensor 13. At time point *t*1, there is a noticeable change in the force signal *f* as the body part 100 starts to engage the probe end 5A and in the temperature signal *T_{IR}* as the body part 100 starts to enter the field of view 13A. As seen, the presence of the body part 100 causes a step change Δ*T*, Δ*f* in the signal *f, T_{IR}.* It should be noted, however, that the step change in temperature may be either positive (as shown), negative or none, depending on the current relation between ambient temperature and skin temperature. Further, in the illustrated example, the temporal variability *var*(*Tₚ*), *var*(*fₚ*) in presence of the body part 100 is larger than the temporal variability *var*(*Tₐ*), *var*(*fₐ*) in absence of the body part 100. The Applicant has realized that one or more characteristics of the signals *f, T_{IR}* in FIG. 2 may be used for discriminating between presence and absence of the body part 100 and thereby enable automated evaluation of the temperature signal *T_{IR}.*

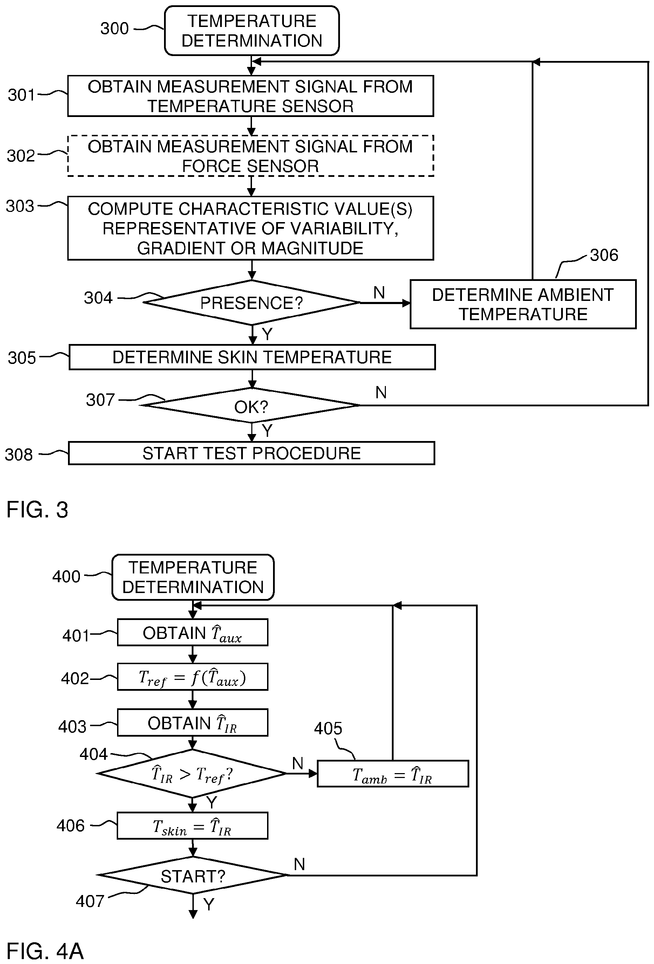

FIG. 3 illustrates a method 300 for temperature determination in accordance with an embodiment of the invention. The method 300 is performed during a preparation phase before initiation of the test procedure for determining VPTs. In this preparation phase, e.g. as disclosed in aforesaid WO2006/046901, it is common practice for the test subject to position the body part 100 on the probe end 5A and apply a longitudinal force within a predefined force range. During the preparation phase, the vibrometer 1 may assist the test subject by providing real-time feedback about the application force via the feedback device 9. The method 300 may be performed in parallel or in series with such a force adjustment procedure. In step 301, first measurement data is obtained from the temperature signal *T_{IR}* of the contactless temperature sensor 13. In step 302, which is optional (see below), second measurement data is obtained from the force signal *f* of the force sensor 7. The first and second measurement data comprises one or more signal values in the respective signal *T_{IR}, f.*

In step 303, one or more characteristic parameters are computed based on the first and/or second measurement data. The respective characteristic parameter is computed to yield one or more characteristic values representative of presence or absence of the body part 100. The characteristic parameter may belong to different categories. In a first category, the characteristic parameter represents the current variability in the temperature signal *T_{IR},* e.g. within a predefined or selected time window Δ*t* (FIG. 2). The variability may represent the signal *T_{IR}* in the time domain ("temporal variability") and may be given by any suitable statistical variability or dispersion metric, such as standard deviation, variance, range or interquartile range, average deviation, mean absolute difference, etc., and normalized variants thereof. Alternatively or additionally, the variability may represent the signal *T_{IR}* in the frequency domain ("spectral variability") and may likewise be given by such a metric of statistical variability. It also conceivable that the spectral variability may be given by the shape of the signal *T_{IR}* in the frequency domain (e.g. a histogram comprising magnitude values as function of frequency), or the average magnitude in a predefined frequency range, or the frequency or frequency range of maximum variability in the signal *T_{IR}.* In a second category, the characteristic parameter represents the current temporal gradient in the temperature signal *T_{IR},* e.g. within the time window Δ*t*. The gradient represents rate of change (signal change per unit time) and may alternatively be denoted "derivative". The computation of the gradient may or may not involve a low-pass filtering of the temperature signal *T_{IR}.* Any conceivable gradient computation function may be used. In a third category, the characteristic parameter represents the current magnitude of the temperature signal *T_{IR},* e.g. given by a single value or by an average of plural signal values, e.g. within the time window Δ*t*.

The foregoing first to third categories of characteristic parameters are equally applicable to and may thus be computed for the force signal *f*.

In step 304, the one or more characteristic parameters are evaluated for detection of presence of the body part 100. Step 304 may involve comparing each computed characteristic parameter to a respective reference, e.g. a range indicative of either presence or absence of the body part. The range may be predefined or determined from the respective signal *T_{IR}, f.* The range may be open-ended or defined by minimum and maximum threshold values. For example, as understood from FIG. 2, presence may be indicated if the current magnitude of the temperature signal *T_{IR}* exceeds a minimum temperature threshold *Tₘᵢₙ,* or if the current magnitude falls within a temperature range between *Tₘᵢₙ* and *Tₘₐₓ.* Correspondingly, presence may be indicated if the current magnitude of the force signal *f* exceeds a minimum force value *fₘᵢₙ.* In another example, presence may be indicated if the current gradient exceeds a gradient threshold, which is set to detect a step change in the temperature signal *T_{IR}* or the force signal *f.* In yet another example, presence may be indicated when the variability exceeds a variability threshold. In the example that the characteristic parameter represents the shape in the frequency domain, the reference in step 304 may comprise one of more shape references, e.g. a respective reference histogram or representative values thereof, and presence may be indicated depending on the amount of deviation between the characteristic parameter and the shape reference(s).

Step 303 may compute two or more characteristic parameters for evaluation by step 304, which determines presence of the body part 100 only if all of the characteristic parameters indicate such presence. The characteristic parameters may be computed to be substantially concurrent in time, e.g. for a common time period (cf. Δ*t* in FIG. 2). However, as exemplified further below, it is equally conceivable that characteristic parameters are computed to represent two separate time points. Depending on the categories of the characteristic parameters, it may be advantageous for step 303 to compute characteristic parameters to represent both of the signals *T_{IR}, f,* such that presence needs to be indicated in both signals before step 304 determines presence of the body part 100. Alternatively or additionally, it may be advantageous for step 303 to compute characteristic parameters in different categories. It is also conceivable that one characteristic parameter is given by a combination of metrics derived from one or both of the signals *T_{IR}, f.* For example, such a composite characteristic parameter may be formed as a (weighted) combination of the current magnitude and the current variability in one of the signals *T_{IR}, f,* or a (weighted) combination of the current variability in each of the signals *T_{IR}, f.*

If step 304 determines presence of the body part 100, the method proceeds to step 305 which sets the skin temperature to the current magnitude in the temperature signal *T_{IR}.* Step 305 may compute the current magnitude for the signal *T_{IR}* or, if the current magnitude was computed by step 303, use the already computed magnitude.

If step 304 fails to determine presence of the body part 100, the method proceeds to step 306 which sets the ambient temperature to the current magnitude in the temperature signal *T_{IR}.* After step 306, the method returns to step 301.

The method 300 in FIG. 3 presumes that the ambient temperature will be determined before the skin temperature. This is a reasonable assumption if the method 300 is performed at startup of vibrometer 1 when the body part 100 is unlikely to be located on the probe 5. Thus, when the skin temperature has been set in step 305, the method proceeds to step 307 which verifies that the current skin temperature falls within a first predefined range, e.g. 27-35°C in accordance with the above-mentioned ISO standard. Step 307 may also verify that the current ambient temperature falls within a second predefined range, e.g. 20-30°C in accordance with the ISO standard. If the verification in step 307 fails, the method returns to step 301. Optionally, if the verification fails or has failed a predefined number of times, the method 300 may inform the test subject via the feedback device 9 and provide instructions for changing the ambient temperature and/or the skin temperature. If the verification succeeds in step 307, the method proceeds to step 308 to start the test procedure, optionally subject to a command entered through the input device 8.

The method 300 facilitates measurement of skin temperature and may be performed during the normal procedure of engaging the body part 100 with the probe end 5A in preparation for the test procedure. The method 300 may be automated and performed without the need to provide dedicated instructions to the test subject and without physical contact between the body part 100 and the sensor 13. The measurement of skin temperature may be even performed without the subject knowing.

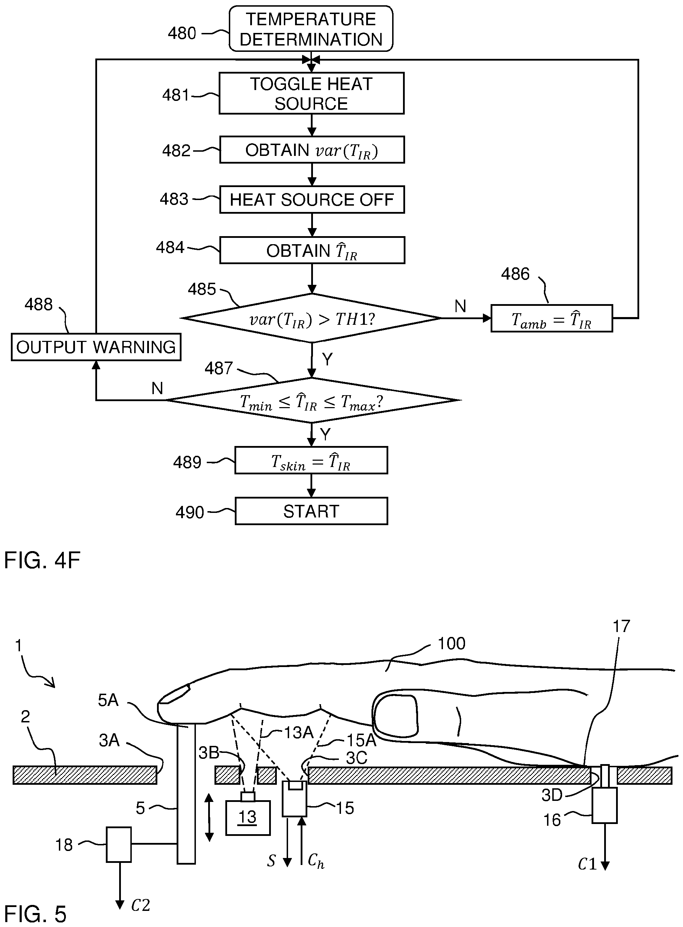

If the characteristic parameter(s) represent both signals *T_{IR}, f,* verification of presence in step 304 produces the additional advantage of verifying that the body part 100 is properly oriented and positioned on the vibrometer 1. This is relevant since vibrometers commonly define a dedicated support surface (cf. 17 in FIG. 5) for the body part 100, to ensure that the VPTs are measured in a consistent and repeatable way. However, conventional vibrometers do not verify the positioning of the body part 100 in relation to the support surface.

Although not shown in FIG. 3, it is conceivable that the control unit continues to measure the skin temperature during the test procedure, i.e. after step 308. This continued measurement of skin temperature may involve steps corresponding to steps 301-305, which verify continued presence of the body part 100 and determine the skin temperature. If the verification fails or if the skin temperature is out of range (cf. step 307), the test procedure may be terminated. It may be noted that different characteristic parameters may be computed during the test procedure compared to the preparation phase before the test procedure. For example, continued presence does not produce a step change (FIG. 2) so the gradient category is not useful as presence indicator at this stage.

In the following, a few non-limiting implementations of the method 300 in FIG. 3 will be described with reference to the flow charts of FIGS 4A-4F. In the following examples, the magnitude of a signal is designated by the accent "^".

The method 400 in FIG. 4A determines presence by use of the magnitude of the temperature signal *T_{IR}* in relation to a reference value *T_{ref}* (magnitude threshold). Step 401 obtains the magnitude *T̂ₐᵤₓ* of the auxiliary temperature signal *Tₐᵤₓ* from sensor 14 (FIG. 1). Step 402 computes the reference value *T_{ref}* as a function of *T̂ₐᵤₓ,* e.g. by setting the reference value *T_{ref}* to or relative to *T̂ₐᵤₓ.* As noted above, the sensor 14 measures the temperature inside the housing 2, which may exceed the ambient temperature in the surroundings of the vibrometer 1 due to heat emitted by the vibrometer electronics. For example, step 402 may set *T_{ref}* by adding a predefined offset to *T̂ₐᵤₓ.* Step 403 obtains the current magnitude *T̂_{IR}* of the temperature signal *T_{IR}.* Step 404 compares the characteristic parameter value *T̂_{IR}* to the reference value *T_{ref}.* If *T̂_{IR}* does not exceed *T_{ref},* the method 400 proceeds to step 405, which sets the ambient temperature *T_{amb}* to *T̂_{IR},* and then returns to step 401. If *T̂_{IR}* exceeds *T_{ref}* in step 404, presence of the body part is detected, and the method proceeds to step 406 which sets the skin temperature *Tₛₖᵢₙ* to *T̂_{IR}.* Step 407 then evaluates if the test procedure may be started, e.g. by verifying that *Tₛₖᵢₙ* and *T_{amb}* are acceptable. If the evaluation in step 407 fails, the method 400 returns to step 401, optionally after presenting the reason for failure on the feedback device 9. If step 407 is successful, the method proceeds towards startup of the test procedure.

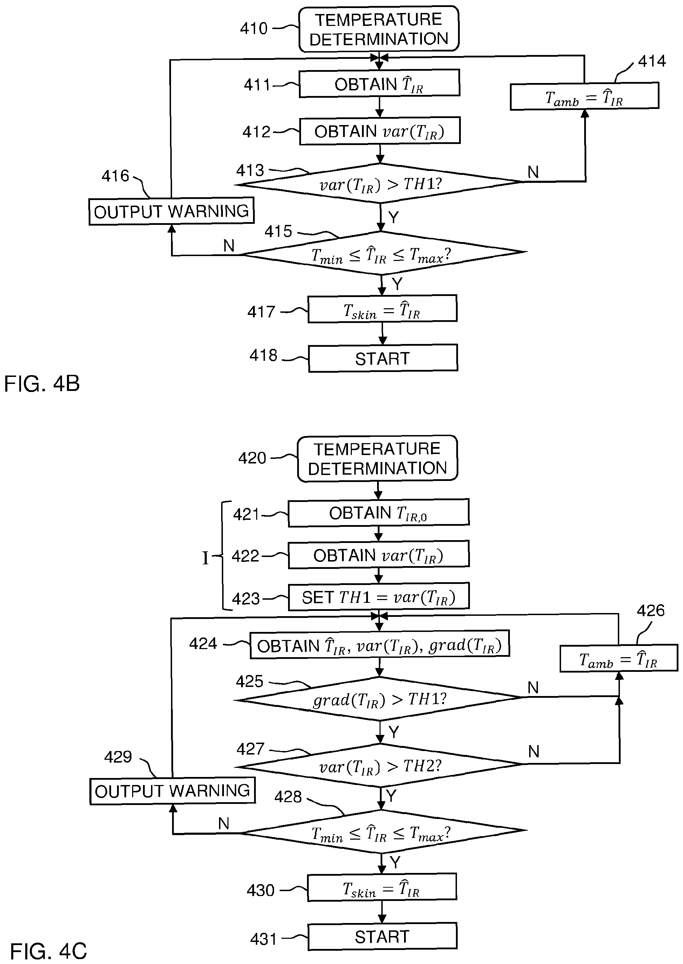

The method 410 in FIG. 4B determines presence by use of the variability in the temperature signal *T_{IR}.* Step 411 obtains the current magnitude *T̂_{IR}* of the temperature signal *T_{IR},* and step 412 obtains the current variability in the temperature signal *T_{IR}.* Step 413 compares this characteristic parameter value, *var*(*T_{IR}*), to a predefined variability threshold TH1, which may be obtained from memory 12 (FIG. 1). If *var*(*T_{IR}*) does not exceed TH1, the method 410 proceeds to step 414, which sets the ambient temperature *T_{amb}* to *T̂_{IR},* and then returns to step 411. If *var*(*T_{IR}*) exceeds TH1, the method proceeds to step 415, which checks if *T̂_{IR}* falls within a predefined range delimited by *Tₘᵢₙ* and *Tₘₐₓ* (cf. FIG. 2). The predefined range may define allowable skin temperatures, e.g. given by the above-mentioned standard. If *T̂_{IR}* is outside the predefined range, step 415 returns to step 411 via step 416, which outputs a warning on the feedback device 9 that the skin temperature is out of range. Otherwise, the method proceeds to step 417, which sets the skin temperature *Tₛₖᵢₙ* to *T̂_{IR},* and then starts the test procedure in step 418.

The method 420 in FIG. 4C determines presence by use of the variability and the gradient in the temperature signal *T_{IR}.* The method includes a reference phase I for determining one or more baseline values. The reference phase I is performed when it can be safely assumed that the body part 100 is absent and includes steps 421-423, e.g. at the onset of the method 420. Step 421 obtains a baseline magnitude *T*_{*IR*,0} given by the magnitude of the temperature signal *T_{IR}.* Step 422 obtains a baseline variability by computing the variability in the temperature signal *T_{IR}.* Reverting to FIG. 2, the baseline variability corresponds to *var(Tₐ*)*.* Step 423 sets a variability threshold TH2 as function of the baseline variability. For example, step 423 may set TH2 equal to or higher than the baseline variability, e.g. by adding a predefined offset. The method then proceeds to step 424, which obtains the current magnitude, *T̂_{IR},* the current variability, *var*(*T_{IR}*), and the current gradient, *grad*(*T_{IR}*), in the temperature signal *T_{IR}.* Step 425 compares the characteristic parameter value *grad*(*T_{IR}*) to a predefined gradient threshold TH1, which may be obtained from memory 12. If *grad*(*T_{IR}*) does not exceed TH1, the method proceeds to step 426, which sets the ambient temperature *T_{amb}* to *T̂_{IR},* and then returns to step 424. If *grad*(*T_{IR}*) exceeds TH1, the method proceeds to step 427 which compares the characteristic parameter value *var*(*T_{IR}*) to the variability threshold TH2. If *var*(*T_{IR}*) does not exceed TH2, the method proceeds to step 426, which sets the ambient temperature *T_{amb}* to *T̂_{IR},* and then returns to step 424. If *var*(*T_{IR}*) exceeds TH2, the method proceeds to step 428, which may correspond to step 415 in FIG. 4B. If the comparison in step 428 fails, the method returns to step 424 via step 429, which may correspond to step 416 in FIG. 4B. Otherwise, the method proceeds to step 430, which sets the skin temperature *Tₛₖᵢₙ* to *T̂_{IR},* and then starts the test procedure in step 431. In a variant, *var*(*T_{IR}*) is obtained in a separate step between steps 425 and 427, so that *grad*(*T_{IR}*) and *var*(*T_{IR}*) are obtained for separate time segments (windows) in the signal *T_{IR}.* Thereby, with reference to FIG. 2, step 425 may detect the step change and step 427 may detect the increased variability subsequent to the step change.

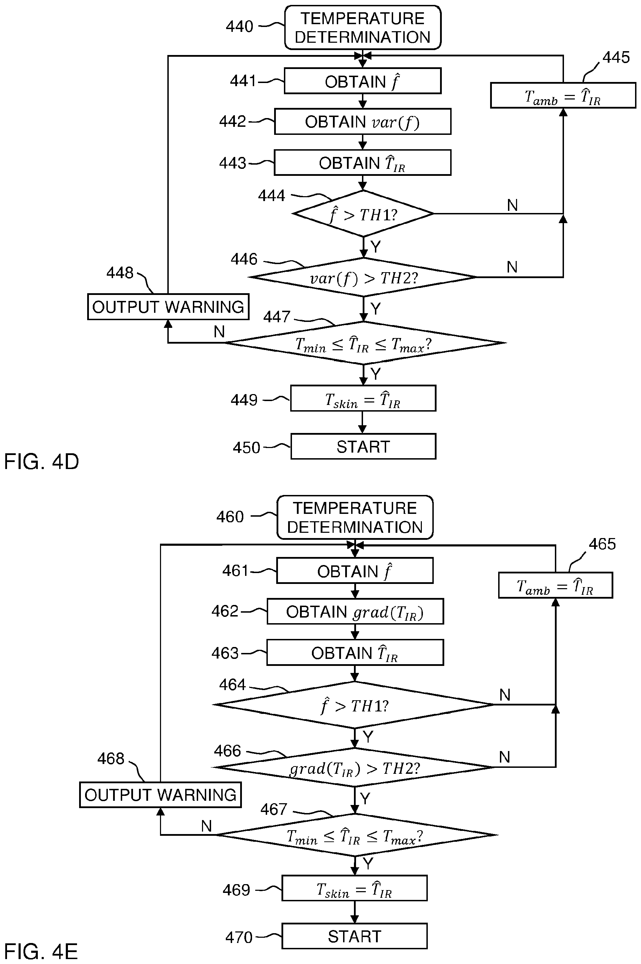

The method 440 in FIG. 4D determines presence by use of the magnitude and the variability in the force signal *f.* Step 441 obtains the current magnitude *f̂* in the force signal *f,* and step 442 obtains the current variability, *var*(*f*), in the force signal *f.* Step 443 obtains the magnitude *T̂_{IR}* of the temperature signal *T_{IR}.* Step 444 compares the characteristic parameter value *f̂* to a predefined magnitude threshold TH1, which may be obtained from memory 12. TH1 may correspond to *fₘᵢₙ* in FIG. 2. If *f̂* does not exceed TH1, the method 440 proceeds to step 445, which sets the ambient temperature *T_{amb}* to *T̂_{IR},* and then returns to step 441. If *f̂* exceeds TH1, the method proceeds to step 446, which compares the characteristic parameter value *var*(*f*) to a predefined variability threshold TH2, which may be obtained from memory 12. Alternatively, TH2 may be obtained during a reference phase in correspondence with TH1 in FIG. 4C (steps 422-423). If *var*(*f*) does not exceed TH2, the method proceeds to step 445, which sets the ambient temperature *T_{amb}* to *T̂_{IR},* and then returns to step 441. If *var*(*f*) exceeds TH2, the method proceeds to step 447, which may correspond to step 415 in FIG. 4B. If the comparison in step 447 fails, the method returns to step 441 via step 448, which may correspond to step 416 in in FIG. 4B. Otherwise, the method proceeds to step 449, which sets the skin temperature *Tₛₖᵢₙ* to *T̂_{IR},* and then starts the test procedure in step 450.

The method 460 in FIG. 4E determines presence by use of the magnitude of the force signal *f* and the gradient in the temperature signal *T_{IR}.* Step 461 obtains the current magnitude *f̂* in the force signal *f,* and step 462 obtains the current gradient, *grad*(*T_{IR}*), in the temperature signal *f.* Step 463 obtains the magnitude *T̂_{IR}* of the temperature signal *T_{IR}.* Step 464 compares the characteristic parameter value *f̂* to a predefined magnitude threshold TH1, which may be obtained from memory 12. TH1 may correspond to *fₘᵢₙ* in FIG. 2. If *f̂* does not exceed TH1, the method proceeds to step 465, which sets the ambient temperature *T_{amb}* to *T̂_{IR},* and then returns to step 461. If *f̂* exceeds TH1, the method proceeds to step 466, which compares the characteristic parameter value *grad*(*T_{IR}*) to a predefined variability threshold TH2, which may be obtained from memory 12. If *grad*(*T_{IR}*) does not exceed TH2, the method proceeds to step 465, which sets the ambient temperature *T_{amb}* to *T̂_{IR},* and then returns to step 461. If *grad*(*T_{IR}*) exceeds TH2, the method proceeds to step 467, which may correspond to step 415 in FIG. 4B. If the comparison in step 467 fails, the method returns to step 461 via step 468, which may correspond to step 416 in FIG. 4B. Otherwise, the method proceeds to step 469, which sets the skin temperature *Tₛₖᵢₙ* to *T̂_{IR},* and then starts the test procedure in step 470.

The method 480 in FIG. 4F uses the variability of the temperature signal *T_{IR}* as a characteristic parameter for detecting presence of the body part and selectively activates a dedicated heat source to enhance the variability and thereby facilitate detection of presence. FIG. 5 shows an embodiment of the vibrometer 1 with such a heat source 15. The heat source 15 is aligned with a third opening 3C in the housing 2 and oriented to emit heat (IR radiation) within the field of view 13A of the sensor 13. Thus, when the heat source 15 is activated and the body part 100 is located within the field of view 13A, the sensor 13 will detect the emitted heat. As indicated in FIG. 5, the control unit 10 may control the activation of the heat source 15 by a control signal *Cₕ.* In step 481, the heat source 15 is repeatedly switched on and off ("toggled") so as to cause an increased variability within the time window Δ*t* (FIG. 2). Concurrently with step 481, step 482 computes the current variability, *var*(*T_{IR}*), in the temperature signal *T_{IR}.* In step 483, the heat source 15 is deactivated and step 484 obtains the current magnitude *T̂_{IR}* of the temperature signal *T_{IR}.* The method then proceeds to step 485, which compares the characteristic parameter value *var*(*T_{IR}*) to a predefined variability threshold TH1, which may be obtained from memory 12. Alternatively, TH1 may be obtained during a reference phase in correspondence with TH1 in FIG. 4C (steps 421-422). If *var*(*T_{IR}*) does not exceed TH1, the method proceeds to step 486, which sets the current ambient temperature *T_{amb}* to *T̂_{IR},* and then returns to step 481. If *var*(*T_{IR}*) exceeds TH1, the method proceeds to step 487, which may correspond to step 415 in FIG. 4B. If the comparison in step 487 fails, the method returns to step 481 via step 488, which may correspond to step 416 in FIG. 4B. Otherwise, the method proceeds to step 489, which sets the skin temperature *Tₛₖᵢₙ* to *T̂_{IR},* and then starts the test procedure in step 490. In an alternative, step 482 determines one or more dominant frequencies in the signal *T_{IR},* and step 485 compares the one or more dominant frequencies to a known toggling frequency, i.e. the frequency at which the heat source 15 is switched on and off. Only if a dominant frequency substantially corresponds to the toggling frequency, step 485 proceeds to step 487.

In a variant of the method 480, step 482 obtains the gradient in the temperature signal *T_{IR}* as the heat source 15 is being switched on, and step 485 compares the gradient to a predefined gradient threshold. In another variant, the step 482 obtains the magnitude of the temperature signal *T_{IR}* when the heat source 15 has been switched on, and step 485 compares this magnitude to a magnitude threshold, which may be predefined or given by the magnitude obtained by step 484. In these variants, step 481 need not repeatedly switch the heat source 15 on and off, since it may be sufficient for step 481 to only switch on the heat source 15 once. Any combination of variability, gradient and magnitude may be evaluated by step 485. It is currently believed that the most robust presence detection is obtained by use of variability.

The evaluation for presence detection may involve signal analysis of one or more further signals in addition to the force signal *f* and/or the temperature signal *T_{IR}.* In the embodiment in FIG. 5, the housing 2 defines a support surface 17 for the body part 100, e.g. for the palm of a hand (as shown) or the heel of a foot. A contact sensor 16 is located to generate an output signal C1 indicative of contact between the body part 100 and the support surface 17. The control unit 10 (FIG. 1) may thus infer that the body part 100 is in contact with the support surface 17 by analysis of the output signal C1. By verifying contact with the support surface 17 and presence on the probe tip 5A (based on the force signal) and/or presence in front of the temperature sensor 13 (based on the temperature signal), it is further ensured that the body part 100 is properly oriented on the vibrometer 1 before the skin temperature is determined and the test procedure is started.

In the example of FIG. 5, the sensor 16 is a mechanical switch, which is aligned with a fourth opening 3D in the housing 2 to be engaged by the body part 100 when the latter is placed on the support surface 17. In a variant (not shown), the sensor 16 is a temperature sensor, where the control unit 10 may infer presence of the body part 100 on the support surface 17 when the magnitude of the output signal C1 is substantially equal (e.g. within ±1-3°C) to the magnitude of the temperature signal *T_{IR},* provided that presence has been detected based on characteristic parameter(s) in accordance with any of the embodiments in FIGS 3-4. In a variant (not shown), the sensor 16 is a contactless capacitive sensor located beneath the support surface 17. In this variant, the opening 3D is optional.

In all embodiments disclosed herein, the force signal *f* may be replaced or supplemented by a contact signal *C*2 indicative of contact between the body part 200 and the probe end 5A. The contact signal *C*2 may be provided by a touch or contact sensor 18 configured to detect that the body part 100 touches the probe end 5A, as indicated in FIG. 5. The sensor 18 may be a conductive or capacitive sensor, which is electrically coupled to at least the probe end 5A. In another variant, the sensor 18 detects touch/contact by sensing changes in a magnetic field.

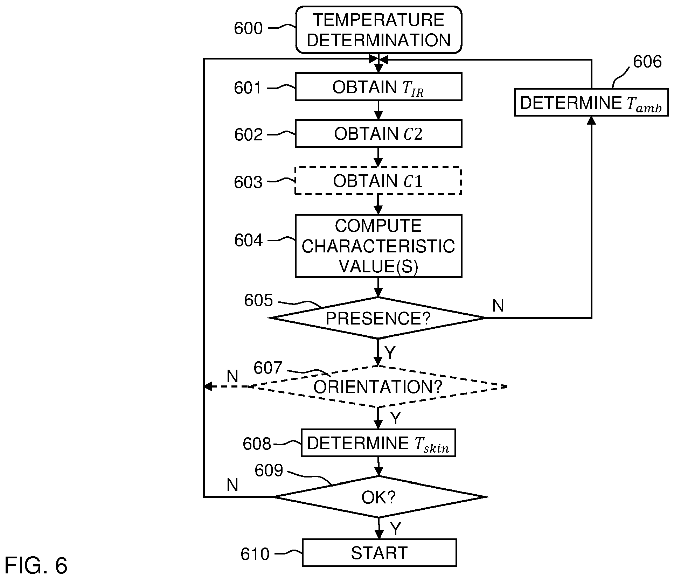

FIG. 6 illustrates a method 600 that detects presence of the body part 100 by use of the contact signal *C*2 of the sensor 18 in FIG. 5, and optionally verifies the orientation of the body part 100 by use of the contact signal *C*1 of the sensor 16 in FIG. 5. Step 601 obtains the temperature signal *T_{IR},* and step 602 obtains the contact signal *C*2. Step 603 is optional and obtains the contact signal *C*1. Step 604 computes one or more characteristic parameters for presence detection based on measurement data in the contact signal *C*2, and optionally based on concurrent measurement data in the temperature signal *T_{IR}.* Step 605 evaluates the characteristic parameter(s) for detection of presence of the body part 100. Steps 604-605 may be performed in correspondence with steps 303-304 of the method 300 in FIG. 3, by replacing the force signal *f* with the contact signal *C*2. If step 605 finds that the body part 100 is absent, the method proceeds to step 606, which sets the ambient temperature *T_{amb}* to *T̂_{IR}* and returns to step 601. If step 605 finds that the body part 100 is present, the method proceeds to step 607 (optional), which evaluates the signal *C*1 for contact between the body part 100 and the support surface 17. If step 607 finds that the body part 100 is not in contact with the surface 17, the method returns to step 601, optionally after instructing the test subject to re-position the body part 100. If step 607 detects contact between the body part 100 and the surface 17, the method concludes that the body part 100 is properly oriented and positioned and proceeds to steps 608-610, which correspond to steps 307-309 in FIG. 3.

In some embodiments, presence of the body part 100 within the field of view 13A of the sensor 13 may be inferred based on the output signal of a radiation detector which is configured to detect the shadow of the body part 100 against ambient radiation, i.e. incoming radiation from the surroundings of the vibrometer 1. In FIG. 5, reference numeral 15 may designate such a "shadow detector" that generates an output signal S. The shadow detector 15 defines a field of view 15A which at least partly overlaps the field of view 13A of the sensor 13. The shadow detector 15 is thus capable of sensing, in zero, one or two dimensions, the partial blocking of the ambient light by the body part 100 when it is located within the field of view 15A. Thus, for improved presence detection, the output signal *S* may be evaluated for detection of the body part 100 in combination with the characteristic parameter(s) of *T_{IR}* and/or *f* and/or *C*2. In one embodiment, the shadow detector 15 is at least partly responsive to radiation in a different wavelength range than the temperature sensor 13, e.g. in the visible wavelength range of approx. 380-780 nm.

In an additional aspect, detection of presence of the body part 100 is primarily based on the output signal *S* of the shadow detector 15, optionally supplemented by evaluation of characteristic parameter(s) in *T_{IR}* and/or *f* and/or *C*2. This additional aspect may be implemented by modifying the method 600 in FIG. 6 so that step 602 obtains the shadow signal *S*, step 604 processes the shadow signal *S* for detection of the body part 100 within the field of view 15A, and step 605 evaluates the outcome of step 604 for presence of the body part 100. Step 604 may optionally compute one or more characteristic parameter(s) in *T_{IR}* and/or *f* and/or *C*2, and step 605 may optionally determine presence of the body part 100 only when both the outcome of step 604 and the characteristic parameter(s) indicate presence of the body part 100.

Further, while operations are depicted in the drawings in a particular order, this should not be understood as requiring that such operations be performed in the particular order shown or in sequential order, or that all illustrated operations be performed, to achieve desirable results. In certain circumstances, parallel processing may be advantageous.

## Claims

1. An apparatus configured to perform a test procedure for measuring vibrotactile perception of a subject, said apparatus comprising:
a vibration probe (5) with an end portion (5A) arranged for engagement by a body part (100) of the subject,
a vibrator (6) coupled to the vibration probe (5) and operable to impart a longitudinal vibration to the vibration probe (5),
a force sensor (7) coupled to the vibration probe (5), said force sensor (7) configured to provide a second output signal (*f*) indicative of an application force onto the end portion (5A),
an input device (8), which allows the subject to signal when the longitudinal vibration of the vibration probe (5) is sensed by the subject,
a control unit (10) configured to perform the test procedure by operating the vibrator (6) to impart the longitudinal vibration to the vibration probe (5) at different intensities at one or more frequencies, and receiving an input signal from the input device (8) indicating when longitudinal vibration is sensed by the subject, the control unit (10) being further configured to monitor, during the test procedure, the second output signal (*f*) to ensure that the application force is adequate,
**characterized in that**
the apparatus further comprises a contactless temperature sensor (13) arranged to face the body part (100) when engaged with the end portion (5A), said contactless temperature sensor (13) configured to provide a first output signal (*T_{IR}*)*,*
the control unit (10) is configured to, before the test procedure and as a function of the first and second output signals (*T_{IR}, f*)*,* compute at least two characteristic parameters indicative of presence or absence of the body part (100),
the control unit (10) is configured to, when presence of the body part (100) is determined based on the at least two characteristic parameters, set a current temperature (*Tₛₖᵢₙ*) of the body part (100) to a current temperature value given by the first output signal (*T_{IR}*) only when all of the characteristic parameters indicate presence of the body part (100), and
the control unit (10) is configured to, when at least one of the at least two characteristic parameters indicates absence of the body part (100) on the end portion (5A), set a current ambient temperature (*T_{amb}*) to the current temperature value given by the first output signal (*T_{IR}*)*.*

2. The apparatus of claim 1, wherein the control unit (10) is configured to determine presence of the body part (100) on the end portion (5A) by evaluating the at least two characteristic parameters in relation to a respective range of values.

3. The apparatus of claim 2, wherein the control unit (10) is configured to compute at least one of the at least two characteristic parameters to represent a temporal gradient in the first or second output signal (*T_{IR}, f),* and evaluate the temporal gradient in relation to a range of gradient values for determination of presence of the body part (100) on the end portion (5A).

4. The apparatus of claim 2 or 3, wherein the control unit (10) is configured to compute at least one of the at least two characteristic parameters to represent a variability in the first or second output signal (*T_{IR}, f),* and evaluate the variability in relation to a variability reference for determination of presence of the body part (100) on the end portion (5A).

5. The apparatus of claim 4, wherein the control unit (10) is configured to, during a reference phase (I) in which the body part (100) is absent from the end portion (5A), compute a baseline temporal variability, and set the variability reference based on the baseline temporal variability.

6. The apparatus of claim 4 or 5, further comprising a heat source (15) which is arranged to generate heat at the body part (100), if the body part (100) is engaged with the end portion (5A), for detection by the contactless temperature sensor (13), said control unit (10) being further configured to cause the heat source (15) to be activated at least once in a predefined time period and compute at least one of the at least two characteristic parameters in the first output signal (*T_{IR}*) when the heat source (15) is activated during said predefined time period, wherein the control unit (10) is further configured to, when presence of the body part (100) is determined based on the at least two characteristic parameters, set the current temperature (*Tₛₖᵢₙ*) of the body part (100) to a current temperature value given by the first output signal (*T_{IR}*) when the heat source is deactivated.

7. The apparatus of any one of claims 2-6, wherein the control unit (10) is further configured to compute at least one of the at least two characteristic parameters to represent a magnitude in the first or second output signal (*T_{IR}, f),* and evaluate the magnitude in relation to a magnitude range for determination of presence of the body part (100) on the end portion (5A).

8. The apparatus of claim 7, comprising a further temperature sensor (14),
wherein the control unit (10) is configured to obtain a temperature value from the further temperature sensor (14) and set the magnitude range based on the temperature value.

9. The apparatus of any preceding claim, further comprising: a support surface portion (17) which is spaced from the vibration probe (5), and a contact sensor (16) arranged at the support surface portion (17) to detect contact between the body part (100) and the support surface portion (17), wherein the control unit (10) is configured to: obtain a third output signal (C1) from the contact sensor (16), and set the current temperature (*Tₛₖᵢₙ*) of the body part (100) to the current temperature value given by the first output signal (*T_{IR}*) only when presence of the body part (100) is determined based on the at least two characteristic parameters and the third output signal (C1) indicates said contact between the body part (100) and the support surface portion (17).

10. A method of preparing an apparatus for measuring vibrotactile perception of a subject, said apparatus comprising a vibration probe (5) having an end portion (5A) arranged for engagement by a body part (100) of the subject, said method being
**characterized by:**
obtaining (301) a first output signal (*T_{IR}*) from a contactless temperature sensor (13) arranged to face the body part (100) when the body part (100) is engaged with the end portion (5A) of the vibration probe (5);
obtaining (302) a second output signal (*f*) from a force sensor (7) coupled to the vibration probe (5), said second output signal (*f*) being indicative of an application force onto the end portion (5A);
computing (303) at least two characteristic parameters of the first and second output signals (*f, T_{IR}*);
evaluating (304) the at least two characteristic parameters for presence of the body part (100);
setting (305), when said evaluating (304) indicates presence of the body part (100), a current temperature (*Tₛₖᵢₙ*) of the body part (100) to a current temperature value given by the first output signal (*T_{IR}*) only when all of the characteristic parameters indicate presence of the body part (100); and
setting (306), when at least one of the at least two characteristic parameters indicates absence of the body part (100) on the end portion (5A), a current ambient temperature (*T_{amb}*) to the current temperature value given by the first output signal (*T_{IR}*).

11. A computer-readable medium comprising computer instructions (21) which, when executed by the control unit (10) in the apparatus according to any one of claims 1-9, cause the control unit (10) to perform the method in accordance with claim 10.

## Patentansprüche

1. Vorrichtung, die zur Durchführung einer Testprozedur zum Messen der vibrotaktiler Wahrnehmung eines Subjekts ausgestaltet ist, wobei die Vorrichtung umfasst:
eine Vibrationssonde (5) mit einem Endabschnitt (5A), der zum Eingriff mit einem Körperteil (100) des Subjekts vorgesehen ist,
einen Vibrator (6), der an die Vibrationssonde (5) gekoppelt ist und funktionell ist, um der Vibrationssonde (5) eine längsgerichtete Vibration zu verleihen,
einen Kraftsensor (7), der an die Vibrationssonde (5) gekoppelt ist, wobei der Kraftsensor (7) ausgestaltet ist, um ein zweites Ausgabesignal (f) bereitzustellen, das eine Applikationskraft auf den Endabschnitt (5A) angibt,
eine Eingabevorrichtung (8), die dem Subjekt das Signalisieren ermöglicht, wann die längsgerichtete Vibration der Vibrationssonde (5) durch das Subjekt gefühlt wird, eine Steuereinheit (10), die ausgestaltet ist, um die Testprozedur durchzuführen, indem der Vibrator (6) betrieben wird, um der Vibrationssonde (5) in unterschiedlichen Intensitäten mit einer oder mehreren Frequenzen die längsgerichtete Vibration zu verleihen, und ein Eingabesignal von der Eingabeeinrichtung (8) empfangen wird, das angibt, wann von dem Subjekt längsgerichtete Vibration gefühlt wird, wobei die Steuereinheit (10) des Weiteren ausgestaltet ist, um während der Testprozedur das zweite Ausgabesignal (f) zu überwachen, um sicherzustellen, dass die Applikationskraft angemessen ist,
**dadurch gekennzeichnet, dass**
die Vorrichtung des Weiteren einen kontaktlosen Temperatursensor (13) umfasst, der vorgesehen ist, um zu dem Körperteil (100) zu weisen, wenn er in Eingriff mit dem Endabschnitt (5A) ist, wobei der kontaktlose Temperatursensor (13) ausgestaltet ist, um ein erstes Ausgabesignal (*T_{IR}*) bereitzustellen,
die Steuereinheit (10) ausgestaltet ist, um vor der Testprozedur und als Funktion des ersten und des zweiten Ausgabesignals (*T_{IR}, f)* mindestens zwei charakteristische Parameter zu berechnen, die Anwesenheit oder Abwesenheit des Körperteils (100) angeben,
die Steuereinheit (10) ausgestaltet ist, um, wenn die Anwesenheit des Körperteils (100) basierend auf den mindestens zwei charakteristischen Parametern bestimmt wird, eine aktuelle Temperatur (*T_{Haut}*) des Körperteils (100) nur dann auf einen aktuellen Temperaturwert zu setzen, der durch das erste Ausgabesignal (*T_{IR}*) gegeben ist, wenn alle der charakteristischen Parameter die Anwesenheit des Körperteils (100) angeben, und
die Steuereinheit (10) ausgestaltet ist, um, wenn mindestens einer der mindestens zwei charakteristischen Parameter die Abwesenheit des Körperteils (100) auf dem Endabschnitt (5A) angibt, eine aktuelle Umgebungstemperatur (*T_{Umg}*) auf den aktuellen Temperaturwert zu setzen, der durch das erste Ausgabesignal (*T_{IR}*) gegeben ist.

2. Vorrichtung nach Anspruch 1, wobei die Steuereinheit (10) ausgestaltet ist, um die Anwesenheit des Körperteils (100) auf dem Endabschnitt (5A) zu bestimmen, indem die mindestens zwei charakteristischen Parameter in Bezug zu einem jeweiligen Wertebereich ausgewertet werden.

3. Vorrichtung nach Anspruch 2, wobei die Steuereinheit (10) ausgestaltet ist, um mindestens einen der mindestens zwei charakteristischen Parameter zu berechnen, so dass ein zeitlicher Gradient in dem ersten oder zweiten Ausgabesignal (*T_{IR}, f)* repräsentiert wird, und den zeitlichen Gradienten in Bezug zu einem Bereich von Gradientenwerten zur Bestimmung der Anwesenheit des Körperteils (100) auf dem Endabschnitt (5A) auszuwerten.

4. Vorrichtung nach Anspruch 2 oder 3, wobei die Steuereinheit (10) ausgestaltet ist, um mindestens einen der mindestens zwei charakteristischen Parameter zu berechnen, so dass eine Variabilität in dem ersten oder zweiten Ausgabesignal (*T_{IR}, f)* repräsentiert wird, und die Variabilität in Bezug zu einer Variabilitätsreferenz zur Bestimmung der Anwesenheit des Körperteils (100) auf dem Endabschnitt (5A) auszuwerten.

5. Vorrichtung nach Anspruch 4, wobei die Steuereinheit (10) ausgestaltet ist, um während einer Referenzphase (I), in der das Körperteil (100) auf dem Endabschnitt (5A) fehlt, eine Basislinie der zeitlichen Variabilität zu berechnen und die Variabilitätsreferenz basierend auf dieser Basislinie der zeitlichen Variabilität zu setzen.

6. Vorrichtung nach Anspruch 4 oder 5, des Weiteren umfassend eine Wärmequelle (15), die vorgesehen ist, um Wärme an dem Körperteil (100) zu generieren, falls das Körperteil (100) in Eingriff mit dem Endabschnitt (5A) ist, um von dem kontaktlosen Temperatursensor (13) detektiert zu werden, wobei die Steuereinheit (10) des Weiteren ausgestaltet ist, um zu bewirken, dass die Wärmequelle (15) mindestens einmal in einer vordefinierten Zeitspanne aktiviert wird, und mindestens einen von den mindestens zwei charakteristischen Parametern in dem ersten Ausgabesignal (*T_{IR}*) zu berechnen, wenn die Wärmequelle (15) während der vordefinierten Zeitspanne aktiviert wird, wobei die Steuereinheit (10) des Weiteren ausgestaltet ist, um, wenn Anwesenheit des Körperteils (100) basierend auf den mindestens zwei charakteristischen Parametern bestimmt wird, die aktuelle Temperatur (*T_{Haut}*) des Körperteils (100) auf einen aktuellen Temperaturwert zu setzen, der durch das erste Ausgabesignal (*T_{IR}*) gegeben ist, wenn die Wärmequelle deaktiviert ist.

7. Vorrichtung nach einem der Ansprüche 2 bis 6, wobei die Steuereinheit (10) des Weiteren ausgestaltet ist, um mindestens einen der mindestens zwei charakteristischen Parameter zu berechnen, so dass eine Größe in dem ersten oder zweiten Ausgabesignal (*T_{IR}, f)* repräsentiert wird, und die Größe in Bezug zu einem Größenbereich zur Bestimmung der Anwesenheit des Körperteils (100) auf dem Endabschnitt (5A) auszuwerten.

8. Vorrichtung nach Anspruch 7, umfassend einen weiteren Temperatursensor (14), wobei die Steuereinheit (10) ausgestaltet ist, um einen Temperaturwert von dem weiteren Temperatursensor (14) zu erhalten und den Grö-ßenbereich basierend auf dem Temperaturwert zu setzen.

9. Vorrichtung nach einem der vorhergehenden Ansprüche, des Weiteren umfassend: einen Trägerflächenabschnitt (17), der von der Vibrationssonde (5) beabstandet ist, und einen Kontaktsensor (16), der an dem Trägerflächenabschnitt (17) angeordnet ist, um Kontakt zwischen dem Körperteil (100) und dem Trägerflächenabschnitt (17) zu detektieren, wobei die Steuereinheit (10) ausgestaltet ist zum: Erhalten eines dritten Ausgabesignals (C1) von dem Kontaktsensor (16), und Setzen der aktuellen Temperatur (*T_{Haut}*) des Körperteils (100) nur dann auf den aktuellen Temperaturwert, der durch das erste Ausgabesignal (*T_{IR}*) gegeben ist, wenn die Anwesenheit des Körperteils (100) basierend auf den mindestens zwei charakteristischen Parametern bestimmt wird und das dritte Ausgabesignal (C1) den Kontakt zwischen dem Körperteil (100) und dem Trägerflächenabschnitt (17) angibt.

10. Verfahren zum Herstellen einer Vorrichtung zum Messen der vibrotaktilen Wahrnehmung eines Subjekts, wobei die Vorrichtung eine Vibrationssonde (5) mit einem Endabschnitt (5A) umfasst, der zum Eingriff mit einem Körperteil (100) des Subjekts vorgesehen ist, wobei das Verfahren **gekennzeichnet ist durch**:
Erhalten (301) eines ersten Ausgabesignals (*T_{IR}*) von einem kontaktlosen Temperatursensor (13), der vorgesehen ist, um zu dem Körperteil (100) zu weisen, wenn das Körperteil (100) in Eingriff mit dem Endabschnitt (5A) der Vibrationssonde (5) ist;
Erhalten (302) eines zweiten Ausgabesignals (f) von einem Kraftsensor (7), der an die Vibrationssonde (5) gekoppelt ist, wobei das zweite Ausgabesignal (f) eine Applikationskraft auf den Endabschnitt (5A) angibt;
Berechnen (303) von mindestens zwei charakteristischen Parametern des ersten und des zweiten Ausgabesignals (*f, T_{IR}*);
Auswerten (304) der mindestens zwei charakteristischen Parameter auf Anwesenheit des Körperteils (100);
wenn die Auswertung (304) Anwesenheit des Körperteils (100) angibt, Setzen (305) einer aktuellen Temperatur (*T_{Haut}*) des Körperteils (100) nur dann auf einen aktuellen Temperaturwert, der **durch** das erste Ausgabesignal (*T_{IR}*) gegeben ist, wenn alle der charakteristischen Parameter die Anwesenheit des Körperteils (100) angeben; und
Setzen (306) einer aktuellen Umgebungstemperatur (*T_{Umg}*) auf den aktuellen Temperaturwert, der **durch** das erste Ausgabesignal (*T_{IR}*) gegeben ist, wenn mindestens einer von den mindestens zwei charakteristischen Parametern die Abwesenheit des Körperteils (100) auf dem Endabschnitt (5A) angibt.

11. Computerlesbares Medium, umfassend Computeranweisungen (21), die bei Ausführung durch die Steuereinheit (10) in der Vorrichtung gemäß einem der Ansprüche 1 bis 9 bewirken, dass die Steuereinheit (10) das Verfahren gemäß Anspruch 10 durchführt.

## Revendications

1. Appareil configuré pour effectuer une procédure de test pour mesurer la perception vibrotactile d'un sujet, ledit appareil comprenant :
une sonde de vibration (5) dotée d'une partie d'extrémité (5A) agencée pour être en prise avec une partie corporelle (100) du sujet,
un vibrateur (6) couplé à la sonde de vibration (5) et capable de transmettre une vibration longitudinale à la sonde de vibration (5),
un capteur de force (7) couplé à la sonde de vibration (5), ledit capteur de force (7) étant configuré pour fournir un second signal de sortie (f) indiquant une force d'application sur la partie d'extrémité (5A),
un dispositif d'entrée (8), qui permet au sujet d'émettre un signal lorsque la vibration longitudinale de la sonde de vibration (5) est détectée par le sujet,
une unité de commande (10) configurée pour exécuter la procédure de test en faisant fonctionner le vibrateur (6) pour transmettre la vibration longitudinale à la sonde de vibration (5) à différentes intensités à une ou plusieurs fréquences, et en recevant un signal d'entrée du dispositif d'entrée (8) indiquant quand la vibration longitudinale est détectée par le sujet, l'unité de commande (10) étant en outre configurée pour surveiller, pendant la procédure de test, le second signal de sortie (f) pour s'assurer que la force d'application est adéquate,
**caractérisé en ce que**
l'appareil comprend en outre un capteur de température sans contact (13) agencé pour faire face à la partie corporelle (100) lorsqu'elle est en prise avec la partie d'extrémité (5A), ledit capteur de température sans contact (13) étant configuré pour fournir un premier signal de sortie (*T_{IR}*),
l'unité de commande (10) est configurée pour, avant la procédure de test et en fonction des premier et deuxième signaux de sortie (*T_{IR}, f),* calculer au moins deux paramètres caractéristiques indicatifs de la présence ou de l'absence de la partie corporelle (100),
l'unité de commande (10) est configurée pour, lorsque la présence de la partie corporelle (100) est déterminée sur la base des au moins deux paramètres caractéristiques, régler une température actuelle (*Tₛₖᵢₙ*) de la partie corporelle (100) à une valeur de température actuelle donnée par le premier signal de sortie (*T_{IR}*) uniquement lorsque tous les paramètres caractéristiques indiquent la présence de la partie corporelle (100), et
l'unité de commande (10) est configurée pour, lorsqu'au moins un des au moins deux paramètres caractéristiques indique l'absence de la partie corporelle (100) sur la partie d'extrémité (5A), régler une température ambiante actuelle (*T_{amb}*) sur la valeur de température actuelle donnée par le premier signal de sortie (*T_{IR}*)*.*

2. Appareil selon la revendication 1, l'unité de commande (10) étant configurée pour déterminer la présence de la partie corporelle (100) sur la partie d'extrémité (5A) en évaluant les au moins deux paramètres caractéristiques par rapport à une plage respective de valeurs.

3. Appareil selon la revendication 2, l'unité de commande (10) étant configurée pour calculer au moins un des au moins deux paramètres caractéristiques pour représenter un gradient temporel dans le premier ou le deuxième signal de sortie (*T_{IR}, f),* et évaluer le gradient temporel par rapport à une plage de valeurs de gradient pour la détermination de la présence de la partie corporelle (100) sur la partie d'extrémité (5A).

4. Appareil selon la revendication 2 ou 3, l'unité de commande (10) étant configurée pour calculer au moins un des au moins deux paramètres caractéristiques pour représenter une variabilité dans le premier ou le deuxième signal de sortie (*T_{IR}, f),* et évaluer la variabilité par rapport à une référence de variabilité pour la détermination de la présence de la partie corporelle (100) sur la partie d'extrémité (5A).

5. Appareil selon la revendication 4, l'unité de commande (10) étant configurée pour, pendant une phase de référence (I) dans laquelle la partie corporelle (100) est absente de la partie d'extrémité (5A), calculer une variabilité temporelle de base, et régler la référence de variabilité sur la base de la variabilité temporelle de base.

6. Appareil selon la revendication 4 ou 5, comprenant en outre une source de chaleur (15) qui est agencée pour générer de la chaleur au niveau de la partie corporelle (100), si la partie corporelle (100) est en prise avec la partie d'extrémité (5A), pour la détection par le capteur de température sans contact (13), ladite unité de commande (10) étant en outre configurée pour faire en sorte que la source de chaleur (15) soit activée au moins une fois dans une période de temps prédéfinie et calculer au moins un des au moins deux paramètres caractéristiques dans le premier signal de sortie (*T_{IR}*) lorsque la source de chaleur (15) est activée au cours de ladite période de temps prédéfinie, l'unité de commande (10) étant en outre configurée pour, lorsque la présence de la partie corporelle (100) est déterminée sur la base des au moins deux paramètres caractéristiques, régler la température actuelle (*Tₛₖᵢₙ*) de la partie corporelle (100) sur une valeur de température actuelle donnée par le premier signal de sortie (*T_{IR}*) lorsque la source de chaleur est désactivée.

7. Appareil selon l'une quelconque des revendications 2 à 6, l'unité de commande (10) étant en outre configurée pour calculer au moins un des au moins deux paramètres caractéristiques pour représenter une amplitude dans le premier ou le deuxième signal de sortie (*T_{IR}, f),* et évaluer l'amplitude par rapport à une plage d'amplitude pour la détermination de la présence de la partie corporelle (100) sur la partie d'extrémité (5A).

8. Appareil selon la revendication 7, comprenant un autre capteur de température (14), l'unité de commande (10) étant configurée pour obtenir une valeur de température à partir de l'autre capteur de température (14) et régler la plage d'amplitude en fonction de la valeur de température.

9. Appareil selon n'importe quelle revendication précédente, comprenant en outre : une partie de surface de support (17) qui est espacée de la sonde de vibration (5), et un capteur de contact (16) agencé au niveau de la partie de surface de support (17) pour détecter le contact entre la partie corporelle (100) et la partie de surface de support (17), l'unité de commande (10) étant configurée pour : obtenir un troisième signal de sortie (C1) en provenance du capteur de contact (16), et régler la température actuelle (*Tₛₖᵢₙ*) de la partie corporelle (100) sur la valeur de température actuelle donnée par le premier signal de sortie (*T_{IR}*) uniquement lorsque la présence de la partie corporelle (100) est déterminée sur la base des au moins deux paramètres caractéristiques et que le troisième signal de sortie (C1) indique ledit contact entre la partie corporelle (100) et la partie de surface de support (17).

10. Procédé de préparation d'un appareil de mesure de la perception vibrotactile d'un sujet, ledit appareil comprenant une sonde de vibration (5) ayant une partie d'extrémité (5A) agencée pour être en prise avec une partie corporelle (100) du sujet, ledit procédé étant **caractérisé par :**
l'obtention (301) d'un premier signal de sortie (*T_{IR}*) en provenance d'un capteur de température sans contact (13) agencé pour faire face à la partie corporelle (100) lorsque la partie corporelle (100) est en prise avec la partie d'extrémité (5A) de la sonde de vibration (5) ;
l'obtention (302) d'un second signal de sortie (f) en provenance d'un capteur de force (7) couplé à la sonde de vibration (5), ledit second signal de sortie (f) étant indicatif d'une force d'application sur la partie d'extrémité (5A) ;
le calcul (303) d'au moins deux paramètres caractéristiques des premier et deuxième signaux de sortie (*f, T_{IR}*)*;*
l'évaluation (304) des au moins deux paramètres caractéristiques de la présence de la partie corporelle (100) ;
le réglage (305), lorsque ladite évaluation (304) indique la présence de la partie corporelle (100), d'une température actuelle (*Tₛₖᵢₙ*) de la partie corporelle (100) à une valeur de température actuelle donnée par le premier signal de sortie (*T_{IR}*) uniquement lorsque tous les paramètres caractéristiques indiquent la présence de la partie corporelle (100) ; et
le réglage (306), lorsqu'au moins un des au moins deux paramètres caractéristiques indique l'absence de la partie corporelle (100) sur la partie d'extrémité (5A), d'une température ambiante actuelle (*T_{amb}*) à la valeur de température actuelle donnée par le premier signal de sortie (*T_{IR}*)*.*

11. Support lisible par ordinateur comprenant des instructions informatiques (21) qui, lorsqu'elles sont exécutées par l'unité de commande (10) dans l'appareil selon l'une quelconque des revendications 1 à 9, amènent l'unité de commande (10) à réaliser le procédé selon la revendication 10.
